(19) Europäisches Patentamt
European Patent Office
Office européen des brevets

(11) Publication number: **0 194 484**
**A2**

(12) # EUROPEAN PATENT APPLICATION

(21) Application number: **86102213.5**

(22) Date of filing: **20.02.86**

(51) Int. Cl.[4]: **C 07 D 211/40**
C 07 D 213/64, A 61 K 31/4-45

(30) Priority **07.03.85 US 709001**

(43) Date of publication of application:
**17.09.86 Bulletin 86/38**

(84) Designated Contracting States:
**DE**

(71) Applicant: **PENNWALT CORPORATION**
**Pennwalt Building Three Parkway**
**Philadelphia Pennsylvania 19102(US)**

(72) Inventor: **Murray, Robert John**
**28 Henderson Drive**
**Penfield N.Y. 14526(US)**

(72) Inventor: **Fedorchuk, Metro**
**19 Musket Lane**
**Pittsford N.Y. 14534(US)**

(74) Representative: **Kraus, Walter, Dr. et al,**
**Patentanwälte Kraus, Weisert & Partner**
**Thomas-Wimmer-Ring 15**
**D-8000 München 22(DE)**

(54) **Synthesis of 1-(2-amino-2-phenylethyl)-2-piperidinones.**

(57) Synthesis of 1-(2-amino-2-phenylethyl)-2-piperidinone and certain derivatives thereof, from 1-phenacyl-2-pyridone and certain derivatives thereof.

EP 0 194 484 A2

Croydon Printing Company Ltd

# Synthesis Of 1-(2-amino-2-phenylethyl)-2-piperidinones

## Background of the Invention

The present invention pertains to the synthesis of 1-(2-amino-2-phenylethyl)-2-piperidinone and certain derivatives thereof, from 1-phenacyl-2-pyridone and certain derivatives thereof.

In H. Mohrle et al, Arch. Pharmaz., 306(5), 325-338 (1973) a process is described for converting 1-phenacyl-2-pyridone to 1-(2-amino-2-phenylethyl)-2-piperidinone by employing three reaction steps. The present application for a patent describes a process for converting 1-phenacyl-2-pyridone to 1-(2-amino-2-phenylethyl)-2-piperidinone by employing two reaction steps: The reaction of 1-phenacyl-2-pyridone with hydroxylamine hydrochloride to form 1-[2-(hydroxyimino)-2-phenylethyl]-2-pyridone, followed by hydrogenation of the latter compound to form 1-(2-amino-2-phenylethyl)-2-piperidinone. The first of these two reaction steps has been reported by E. S. Hand and W. W. Paudler, J. Org. Chem., volume 43, p. 658-663 (1978). To Applicants' knowledge, the combination of the two reaction steps to prepare 1-(2-amino-2-phenylethyl)-2-piperidinone has not been reported. It will be evident, from the date disclosed here that the 2-step process which is the present invention results in a yield of 1-(2-amino-2-phenylethyl)-2-pyridone greater than that reported by H. Mohrle et al, for the 3-step process.

## Brief Summary of the Invention

The invention is a process which comprises the steps of:

(a) contacting hydroxylamine with a pyridone of the formula

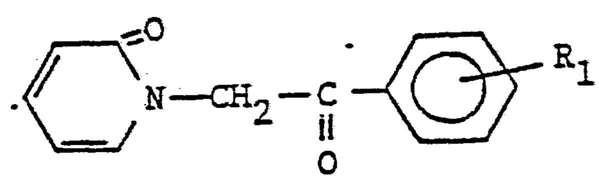

to obtain an oxime of the formula,

 

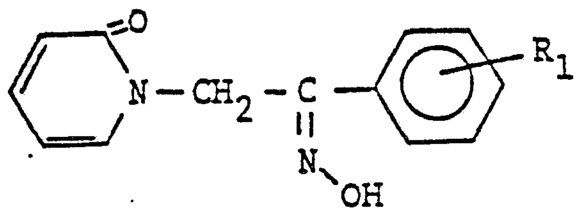

and

(b) reducing said oxime to form a piperidinone of the formula

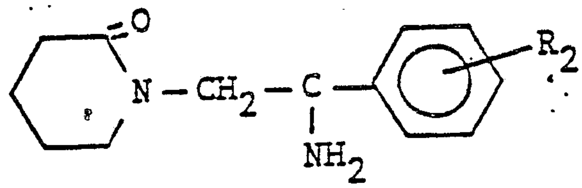

wherein $R_1$ is H, lower alkyl, $NO_2$, lower alkylamino, OH, lower alkoxy or halogen, and $R_2$ is H, lower alkyl, $NH_2$, lower alkylamino, OH, lower alkoxy, or halogen, provided that

(1) $R_2$ is the same as $R_1$ when $R_1$ is not $NO_2$, and

(2) $R_2$ is $NH_2$ when $R_1$ is $NO_2$.

The invention is also the process consisting of step (b) above.

As used herein, the term "lower alkyl" refers to an alkyl group, straight or branched, containing four or less carbon atoms. A lower alkylamino group is one in which the alkyl moiety is straight or branched and contains four or less carbon atoms. A lower alkoxy group is straight or branched and contains four or less carbon atoms.

## Detailed Description

(1) <u>Formation of 1-[2-(hydroxyimino)-2-phenylethyl]-2-pyridone and derivatives thereof from 1-phenacyl-2-pyridone and derivatives thereof.</u>

The oxime, 1-[2-(hydroxyimino)-2-phenylethyl]-2-pyridone, or derivatives thereof in which a nuclear hydrogen of the

phenyl moiety is substituted with a lower alkyl, $NH_2$, lower alkylamino, $NO_2$, OH, lower alkoxy or a halogen, can be formed by reacting 1-phenacyl-2-pyridone or the corresponding derivative thereof, with an excess of hydroxylamine hydrochloride in an alcoholic solvent in the presence of a base at a temperature of at least 20°C but preferably at the reflux temperature of the solvent. The cooled reaction is diluted with water to precipitate the oxime and the resulting solid is filtered off and dried. Suitable solvents are lower alcohols, preferably methanol, ethanol and propanol. Suitable bases are pyridine, sodium acetate and the like. When pyridine is used, a molar excess of 5%-30% of pyridine and hydroxylamine hydrochloride are preferred. The oximes produced are mixtures of their *syn* and *anti* forms. It is not necessary to separate the isomers since both of them afford the same product upon hydrogenation as described below.

(2) Formation of 1-(2-amino-2-phenylethyl)-2-piperidinone and derivatives thereof from 1-[2-(hydroxyimino)-2-phenylethyl]-2-pyridone and derivatives thereof.

The piperidinone, 1-(2-amino-2-phenylethyl)-2-piperidinone or derivatives thereof in which a nuclear hydrogen of the phenyl group is substituted with a lower alkyl, $NH_2$, lower alkylamino, OH, lower alkoxy or a halogen, can be formed by hydrogenating 1-[2-(hydroxyimino)-2-phenylethyl]-2-pyridone or the corresponding derivative thereof over one of the more active noble metal

catalysts, such as palladium or platinum, at a hydrogen pressure of 1-10 atmospheres and at ambient or higher temperatures until 4 molar equivalents of hydrogen are absorbed. Preferred reaction temperatures are in the range 20°C to 80°C, but higher temperatures up to the boiling point of the solvent under the existing pressure conditions can be used when that boiling point exceeds 80°C. In the range 20°C to 80°C, the reaction rate has been found to increase with increasing temperature. If the hydrogenation is performed on the 1-[2-(hydroxyimino)-2-phenylethyl]-2-pyridone derivative in which a nuclear hydrogen of the phenyl group is substituted with $NO_2$, the reaction will absorb an additional 3 molar equivalents of hydrogen and yield the 1-(2-amino-2-phenylethyl)-2-piperidinone derivative in which a nuclear hydrogen of the phenyl group is substituted with $NH_2$. At 50°C, the reaction requires 6-13 hours.

The reaction may be run under neutral or acidic conditions equally well. It may be run in protic or aprotic solvents. Protic solvents such as lower alcohols (particularly methanol, ethanol or propanol) are preferred and the acid is preferably hydrochloric acid although other inorganic acids can be used. On completion of hydrogen absorption, the catalyst is removed by filtration; subsequent evaporation of the filtrate affords the piperidinone. Under acidic conditions, the amine salt of the piperidinone is obtained and the base may be recovered by neutralization with an

aqueous organic base and subsequent extraction of the amine into an organic solvent such as chloroform, methylene chloride, toluene or diethylether.

## Illustrative Examples

### Example 1

Synthesis of 1-[2-(hydroxyimino)-2-phenylethyl]-2-pyridone (First Example)

To a stirred mixture of 11.6 g (0.054 mol) of 1-phenacyl-2-pyridone, 4.8 g (0.069 mol) of hydroxylamine hydrochloride and 50 ml of absolute ethanol was added 5.6 ml (0.069 mol) of pyridine. The mixture was refluxed (about 78°C) with stirring for 1.5 hrs. at which point thin layer chromatography indicated that the reaction was complete. The reaction mixture was cooled and, when crystallization began, 75 ml of $H_2O$ was added. Stirring was continued for 1 hr. The product was isolated by filtration and washed with 30 ml of 30% ethanol and then with $H_2O$. The product was air-dried to yield 10.2 g (82%) of the title compound, mp 162-170°C.

### Example 2

Synthesis of 1-[2-(hydroxyimino)-2-phenylethyl]-2-pyridone (Second Example)

To 1-phenacyl-2-pyridone (634.4 g, 2.97 mol) partially dissolved in 7.41 of absolute ethanol was added hydroxylamine

hydrochloride (518.7 g, 7.46 mol) followed by 1.5 liters of pyridine. The clear, nearly colorless solution was heated at reflux for 2 hrs., allowed to cool to room temperature and the solvents were removed under reduced pressure. The residual light-brown paste was stirred with 6.25 liters of ice/$H_2O$ and the resulting off-white solid was filtered off, washed well with cold water and dried in vacuo; the yield of off-white crystals was 595.8 g (87.9%), mp 172-174°C.

## Example 3

### Synthesis of 1-(2-amino-2-phenylethyl)-2-piperidinone

A Paar hydrogenation apparatus was charged with 6 g (0.262 mol) of 1-[2-(hydroxyimino)-2-phenylethyl]-2-pyridone (synthesized by the method of Example 1), 40 ml of methanol and 0.6 g of 5% palladium on carbon catalyst. The mixture was hydrogenated under 4.45 bar of hydrogen while maintaining the reaction mixture at 50°C. Hydrogen uptake was complete within 6 hrs. The catalyst was filtered off and the solvent was removed under reduced pressure. The residual oil solidified readily to give the title compound, as evidenced by thin layer chromatography, in a yield of 5.68g (100%), mp 72-73.5°C (n-hexane).

### Synthesis of Substituted Compounds

The synthesis of 1-(2-amino-2-phenylethyl)-2-piperidinone derivatives in which a nuclear hydrogen of the phenyl moiety is substituted with a lower alkyl, $NH_2$, lower alkylamino, OH,

lower alkoxy, or halogen, can be formed by appropriate substitution of reagents in Examples 1, 2 and 3. For example, in either Example 1 or Example 2, following essentially the same procedure but substituting,

1-p-methylphenacyl-2-pyridone,

1-p-chlorophenacyl-2-pyridone,

1-p-nitrophenacyl-2-pyridone,

1-p-methoxyphenacyl-2-pyridone,

1-o-methoxyphenacyl-2-pyridone,

1-m-methoxyphenacyl-2-pyridone,

1-m-(methylamino)phenacyl-2-pyridone,

1-m-hydroxyphenacyl-2-pyridone and

1-m-aminophenacyl-2-pyridone, for the

1-phenacyl-2-pyridone results in the formation of the following compounds respectively,

1-[2-(hydroxyimino)-2-(p-methylphenyl)ethyl]-2-pyridone,

1-[2-(hydroxyimino)-2-(p-chlorophenyl)ethyl]-2-pyridone,

1-[2-(hydroxyimino)-2-(p-nitrophenyl)ethyl]-2-pyridone,

1-[2-(hydroxyimino)-2-(p-methoxyphenyl)ethyl]-2-pyridone,

1-[2-(hydroxyimino)-2-(o-methoxyphenyl)ethyl]-2-pyridone,

1-[2-(hydroxyimino)-2-(m-methoxyphenyl)ethyl]-2-pyridone,

1-[2-(hydroxyimino)-2-[m-(methylamino)phenyl]ethyl]-2-pyridone,

1-[2-(hydroxyimino)-2-(m-hydroxyphenyl)ethyl]-2-pyridone,

and

1-[2-(hydroxyimino)-2-(m-aminophenyl)ethyl]-2-pyridone.

In Example 3, following essentially the same procedure but substituting,

1-[2-(hydroxyimino)-2-(p-methylphenyl)ethyl]-2-pyridone,

1-[2-(hydroxyimino)-2-(p-chlorophenyl)ethyl]-2-pyridone,

1-[2-(hydroxyimino)-2-(p-nitrophenyl)ethyl]-2-pyridone,

1-[2-(hydroxyimino)-2-(p-methoxyphenyl)ethyl]-2-pyridone,

1-[2-(hydroxyimino)-2-(o-methoxyphenyl)ethyl]-2-pyridone,

1-[2-(hydroxyimino)-2-(m-methoxyphenyl)ethyl]-2-pyridone,

1-[2-(hydroxyimino)-2-[m-(methylamino)phenyl]ethyl]-2-pyridone,

1-[2-(hydroxyimino)-2-(m-hydroxyphenyl)ethyl]-2-pyridone,

and

1-[2-(hydroxyimino)-2-(m-aminophenyl)ethyl]-2-pyridone

for the 1-[2-(hydroxyimino)-2-phenylethyl]-2-pyridone results in the formation of the following compounds respectively,

1-[2-amino-2-(p-methylphenyl)ethyl]-2-piperidinone,

1-[2-amino-2-(p-chlorophenyl)ethyl]-2-piperidinone,

1-[2-amino-2-(p-aminophenyl)ethyl]-2-piperidinone,

1-[2-amino-2-(p-methoxyphenyl)ethyl]-2-piperidinone,

1-[2-amino-2-(o-methoxyphenyl)ethyl]-2-piperidinone,

1-[2-amino-2-(m-methoxyphenyl)ethyl]-2-piperidinone,

1-[2-amino-2-[m-(methylamino)phenyl]ethyl]-2-piperidinone,

1-[2-amino-2-(m-hydroxyphenyl)ethyl]-2-piperidinone, and 1-[2-amino-2-(o-aminophenyl)ethyl]-2-piperidinone.

Utility:

The processes of this invention can be used to synthesize 1-(2-amino-2-phenylethyl)-2-piperidinone and derivatives thereof, which are useful as intermediates in the synthesis of 8-substituted 2,3,5,6,7,8-hexahydro-2-phenylimidazo[1,2-a]pyridine hydrochloride and derivatives thereof, compounds which have been disclosed in U.S. patent application number 518,514, of T. A. Davidson et al, [filed on July 29, 1983, Notice of Allowance mailed on Octover 22, 1984, Issue Fee paid on January 3, 1985] as having immunomodulatory and related activities.

The synthesis of 8-substituted 2,3,5,6,7,8-hexahydro-2-phenylimidazo[1,2-a]pyridine (or derivatives thereof) can be accomplished by converting 1-(2-amino-2-phenylethyl)-2-piperidinone (or derivatives thereof as defined in the present invention) to 2,3,5,6,7,8-hexahydro-2-phenylimidazo[1,2-a] pyridine (or derivatives thereof) and then, by the procedures of U.S. Application 518.514, converting that pyridine to an 8-substituted 2,3,5,6,7,8-hexahydro-2-phenylimidazo[1,2-a] pyridine (or derivative thereof). The conversion of 1-(2-amino-2-phenylethyl)-2-piperidone to 2,3,5,6,7,8-hexahydro-2-phenylimidazo[1,2-a]pyridine has been accomplished by Applicant

by the following procedure, similar but not identical to that of Mohrle et al:

To 283.8 g (1.3 mol) of 1-(2-amino-2-phenylethyl)-2-piperidinone dissolved in 2.7 liter of xylene was added 3.26 g of p-toluenesulfonic acid, and the solution was heated at reflux using a Dean-Stark Trap for the removal of $H_2O$ as it was formed. After 4 hours the solution was allowed to cool and the solvent was evaporated off at reduced pressure. The residual oil was kugelrohr distilled (bath temp. 135-170°C/0.1 mm) to afford 223.9 g (86%) of the bicyclic amidine as a nearly colorless oil which solidified on standing. [In another modification of this reaction the xylene solution is washed with water to remove the p-toluenesulfonic acid and then evaporated to give the product as an oil which can be used in subsequent reactions without purification.]

What Is Claimed Is:

1. A process which comprises the steps of:

(a) contacting hydroxylamine with a pyridone of the formula

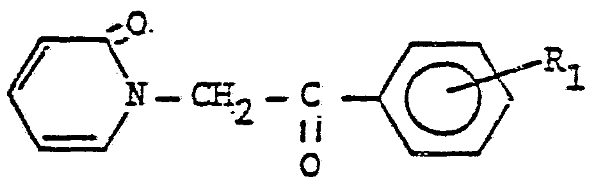

to form an oxime of the fomula

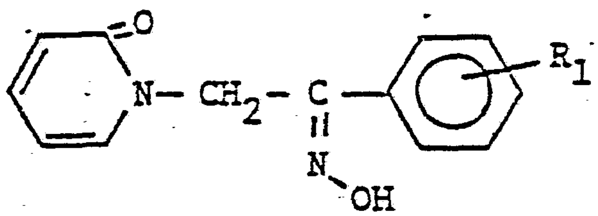

and

(b) reducing said oxime to form a piperidinone of the formula

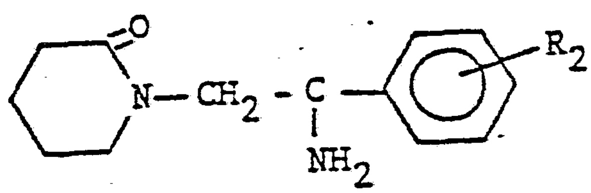

wherein $R_1$ is H, lower alkyl, $NO_2$, lower alkylamino, OH, lower alkoxy or halogen, and $R_2$ is H, lower alkyl, $NH_2$, lower alkylamino, OH, lower alkoxy or halogen, provided that

(1) $R_2$ is the same as $R_1$ when $R_1$ is not $NO_2$, and

(2) $R_2$ is $NH_2$ when $R_1$ is $NO_2$.

2. A process which comprises reducing an oxime of the formula

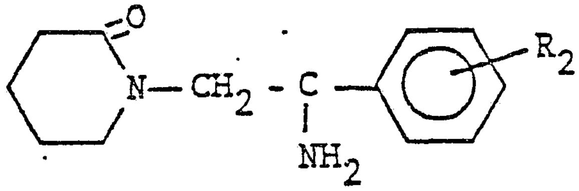

wherein $R_1$ is defined as in Claim 1 to form a piperidinone of the formula

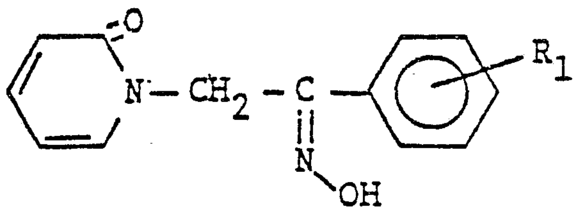

wherein $R_2$ is defined as in Claim 1.

3. A process as defined in Claim 1 wherein $R_1$ and $R_2$ are each H.

4. A process as defined in Claim 2 wherein $R_1$ and $R_2$ are each H.

5. A process as defined in Claim 1 wherein in step (a) there is an excess of hydroxylamine and the reaction is performed in an alcoholic solvent in the presence of a base at a temperature between 20°C and the reflux temperature of the solvent and in step (b) the reaction is performed in the temperature range 20°C to the boiling point of the solvent under the existing pressure conditions, at a hydrogen pressure of 1-10 atmospheres in the presence of an active noble metal catalyst.